# EUROPEAN PATENT APPLICATION

(11) **EP 4 382 022 A1**
(43) Date of publication of application: **12.06.2024**
(21) Application number: 22903330.3
(22) Date of filing: 02.12.2022
(51) Int. Cl.: A61B 1/005, A61B 1/00

(54) **ENDOSCOPE KNOB DEVICE, ENDOSCOPE HANDLE, AND ENDOSCOPE**

(30) Priority: 07.12.2021 CN 202111487180
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); TAN, Xiaofeng, Guangzhou, Guangdong 510000 (CN)
(74) Representative: Murgitroyd & Company
(86) International application number: PCT/CN2022/136209
(87) International publication number: WO 2023/103908

(57) **Abstract**

An endoscope knob device (100), an endoscope handle, and an endoscope. The endoscope knob device (100) includes a first cable pulley (120) defining a first perforation (121), a first matching portion (122), and a first latching portion (123); a second cable pulley (130) defining a second matching portion (132) and a second latching portion (133); and a first knob (140) and a second knob (150) which are sleeved over the first cable pulley (120) and the second cable pulley (130), respectively. The first knob (140) includes a third matching portion (141) and a third latching portion (142) that matches with the first latching portion (123). The third matching portion (141) cooperates with the first matching portion (122), so that the first knob (140) drives the first cable pulley (120) to rotate. The second knob (150) includes a fourth matching portion (151) and a fourth latching portion (152) that matches with the second latching portion (133). The fourth matching portion (151) cooperates with the second matching portion (132), so that the second knob (150) drives the second cable pulley (130) to rotate. The endoscope knob device (100) has an ingeniously designed overall structure in which the first knob (140) and the second knob (150) only need to be sleeved to complete the installation, which is convenient for the assembly of the product, improving the assembly efficiency, thereby effectively reducing the production cost.

## Description

### TECHNICAL FIELD

The present application relates to the technical field of medical devices, and particularly to an endoscope knob device, an endoscope handle, and an endoscope.

### BACKGROUND

Endoscopes are inspection instruments that integrate traditional optics, ergonomics, precision machinery, modern electronics, mathematics, software, etc. It includes image sensors, optical lenses, light sources, mechanical devices, etc. As an important auxiliary medical instrument for examining lesions in the internal organs of the human body, the endoscope is widely used in various surgical operations. At present, most endoscope products are reusable and require strict disinfection procedures after each use. A traditional endoscope includes a handle, an insertion tube, a bending section, a distal tip, and a host. The handle is integrated with structures such as functional buttons, air and water valves, negative pressure valves, three-way valves, etc.

In order to expand the observation range, the bending section is controlled to bend in four directions through a knob device, enabling the endoscope to observe in four directions. However, due to the structural design defects of the existing knob device, it is difficult to assemble, which not only increases the production cost but also seriously affects the assembly efficiency of the endoscope.

### SUMMARY

In view of this, it is necessary to provide an endoscope knob device, an endoscope handle, and an endoscope with ingenious design and low cost, in addition, it is also convenient to assemble and conducive to improving assembly efficiency.

An endoscope knob device includes a housing, a first cable pulley, a second cable pulley, a first knob, and a second knob. The housing defines a mounting hole that communicates inside and outside of the housing. The first cable pulley defines a first perforation. The first cable pulley is rotatably mounted in the housing and extends out of the housing through the mounting hole. The first cable pulley includes a first matching portion and a first latching portion, which are located outside the housing. The second cable pulley is mounted in the housing and extends out of the housing through the first perforation. The second cable pulley includes a second matching portion and a second latching portion, which are located outside the housing. The first knob and the second knob are both located outside the housing. The first knob and the second knob are sleeved over the first cable pulley and the second cable pulley, respectively. The first knob includes a third matching portion and a third latching portion latch-fitted with the first latching portion. The third matching portion cooperates with the first matching portion, so that the first knob drives the first cable pulley to rotate. The second knob includes a fourth matching portion and a fourth latching portion latch-fitted with the second latching portion. The fourth matching portion cooperates with the second matching portion, so that the second knob drives the second cable pulley to rotate.

According to the endoscope knob device, during assembly, the first cable pulley is rotatably mounted inside the housing and extends out of the housing. Then, the first knob is sleeved over the portion of the first cable pulley that extends out of the housing. The first matching portion and the third matching portion are matched, and the first latching portion and the third latching portion are latch-fitted, such that the first cable pulley and the first knob are securely connected, ensuring that the first cable pulley is stably mounted in the housing. In addition, with the first matching portion and the third matching portion, the first knob can drive the first cable pulley to rotate, so as to stably control the bending of the bending section. Similarly, the second cable pulley is rotatably mounted inside the housing, passes through the first perforation, and extends out of the housing. Then, the second knob is sleeved over the portion of the second cable pulley that extends out of the housing. The second matching portion and the fourth matching portion are matched, and the second latching portion and the fourth latching are latch-fitted, such that the second cable pulley and the second knob are securely connected, ensuring that the second cable pulley is stably mounted in the housing. In addition, with the second matching portion and the fourth matching portion, the second knob can drive the second cable pulley to rotate, so as to stably control the bending of the bending section. The whole structure is ingeniously designed, the first knob and the second knob only need to be sleeved to complete the installation, which is convenient for the assembly of the product, improving the assembly efficiency, thereby effectively reducing the production cost.

In one of the embodiments, the endoscope knob device further includes a first bracket mounted in the housing, and the first bracket is sleeved on the second cable pulley and covers the first cable pulley.

In one of the embodiments, the endoscope knob device further includes a first sealing ring arranged between the first bracket and the second cable pulley.

In one of the embodiments, the endoscope knob device further includes a second sealing ring arranged between the first cable pulley and the housing.

In one of the embodiments, a first limiting portion and a second limiting portion are disposed at opposite ends of the first bracket, respectively. The first cable pulley includes a third limiting portion, which cooperates with the first limiting portion to limit a rotation angle of the first cable pulley. The second cable pulley includes a fourth limiting portion, which cooperates with the second limiting portion to limit a rotation angle of the second cable pulley.

In one of the embodiments, at least two first latching portions and at least two third latching portions are configured, the at least two first latching portions are arranged at intervals along a circumferential direction of the first cable pulley, and the at least two third latching portions and the at least two first latching portions are arranged in one-to-one correspondence.

In one of the embodiments, each first latching portion includes a first latching block. Each third latching portion includes a first latching groove that matches with the first latching block. One of the first latching blocks includes a first alignment portion, and a wall of one of the first latching grooves includes a second alignment portion that matches with the first alignment portion.

In one of the embodiments, at least two second latching portions and at least two fourth latching portions are configured. The at least two second latching portions are arranged at intervals along a circumferential direction of the second cable pulley, and the at least two fourth latching portions and the at least two second latching portions are arranged in one-to-one correspondence.

In one of the embodiments, each second latching portion includes a second latching groove. Each fourth latching portion includes a second latching block that matches with the second latching groove. A wall of one of the second latching grooves includes a third alignment portion, and one of the second latching blocks includes a fourth alignment portion that matches with the third alignment portion.

In one of the embodiments, at least two first matching portions and at least two third matching portions are configured. The at least two first matching portions are arranged at intervals along a circumferential direction of the first cable pulley. The at least two third matching portions and the at least two first matching portions are arranged in one-to-one correspondence.

In one of the embodiments, each first matching portion includes a first matching groove. Each third matching portion includes a first matching block that matches with the first matching groove. The first matching groove extends along an axial direction of the first cable pulley.

In one of the embodiments, at least two second matching portions and at least two fourth matching portions are configured, the at least two second matching portions are arranged at intervals along a circumferential direction of the second cable pulley, and the at least two second matching portions and the at least two fourth matching portions are arranged in one-to-one correspondence.

In one of the embodiments, each second matching portion includes a second matching groove, each fourth matching portion includes a second matching block that matches with the second matching groove, and the second matching groove extends along an axial direction of the second cable pulley.

In one of the embodiments, at least two first latching portions and at least two third latching portions are configured. The at least two first latching portions are arranged at intervals along a circumferential direction of the first cable pulley. The at least two third latching portions and the at least two first latching portions are arranged in one-to-one correspondence. At least two first matching portions and at least two third matching portions are configured. The at least two first matching portions are arranged at intervals along a circumferential direction of the first cable pulley. The at least two third matching portions and the at least two first matching portions are arranged in one-to-one correspondence. The first knob defines a first through hole for the first cable pulley to pass through. The at least two third matching portions are arranged at intervals and surround the first through hole. The at least two third latching portions are arranged at intervals and surround the first through hole.

In one of the embodiments, a first protrusion is disposed on a side of the first knob facing the housing, surrounding the first through hole. The first protrusion is configured to cooperate with a first braking assembly for braking.

In one of the embodiments, at least two second latching portions and at least two fourth latching portions are configured. The at least two second latching portions are arranged at intervals along a circumferential direction of the second cable pulley. The at least two fourth latching portions and the at least two second latching portions are arranged in one-to-one correspondence. At least two second matching portions and at least two fourth matching portions are configured. The at least two second matching portions are arranged at intervals along a circumferential direction of the second cable pulley. The at least two second matching portions and the at least two fourth matching portions are arranged in one-to-one correspondence. The second knob defines a second through hole for the second cable pulley to pass through. The at least two fourth matching portions are arranged at intervals and surround the second through hole. The at least two fourth latching portions are arranged at intervals and surround the second through hole.

In one of the embodiments, a mounting groove for a second braking assembly to be mounted is defined on a side of the second knob away from the first knob. A second protrusion is disposed on a wall of the mounting groove, surrounding the second through hole. The second protrusion is configured to cooperate with the second braking assembly for braking.

In one of the embodiments, the endoscope knob device further includes a a second bracket mounted in the housing, the second cable pulley defines a second perforation, and the second bracket covers the second cable pulley and extends out of the housing through the second perforation to connect to a second braking assembly.

In one of the embodiments, the first wire cable pulley includes a first winding portion and a first mounting portion disposed on the first winding portion. The first perforation extends throughout the first winding portion and the first mounting portion. The first winding portion is located in the housing. The first mounting portion passes through the mounting hole. The first matching portion and the first latching portion are arranged on the first mounting portion.

In one of the embodiments, the second wire cable pulley includes a second winding portion and a second mounting portion disposed on the second winding portion. The second winding portion is located in the housing. The second mounting portion passes through the first perforation. The second matching portion and the second latching portion are arranged on the second mounting portion.

An endoscope handle includes a first braking assembly, a second braking assembly and the endoscope knob device described in any one of the above embodiments. The first braking assembly is configured to brake the first knob or the first cable pulley, and the second braking assembly is configured to brake the second knob or the second cable pulley.

The above-mentioned endoscope handle employs the above endoscope knob device. During the assembly process, the first cable pulley is rotatably mounted inside the housing and extends out of the housing. Then, the first knob is sleeved over the portion of the first cable pulley that extends out of the housing. The first matching portion and the third matching portion are matched, and the first latching portion and the third latching portion are latch-fitted, such that the first cable pulley and the first knob are securely connected, ensuring that the first cable pulley is stably mounted in the housing. In addition, with the first matching portion and the third matching portion, the first knob can drive the first cable pulley to rotate, so as to stably control the bending of the bending section. Similarly, the second cable pulley is rotatably mounted inside the housing, passes through the first perforation, and extends out of the housing. Then, the second knob is sleeved over the portion of the second cable pulley that extends out of the housing. The second matching portion and the fourth matching portion are matched, and the second latching portion and the fourth latching are latch-fitted, such that the second cable pulley and the second knob are securely connected, ensuring that the second cable pulley is stably mounted in the housing. In addition, with the second matching portion and the fourth matching portion, the second knob can drive the second cable pulley to rotate, so as to stably control the bending of the bending section. The whole structure is ingeniously designed, the first knob and the second knob only need to be sleeved to complete the installation, which is convenient for the assembly of the product, improving the assembly efficiency, thereby effectively reducing the production cost.

An endoscope is provided, which includes the above-mentioned endoscope handle.

The above-mentioned endoscope employs the above endoscope handle. During the assembly process, the first cable pulley is rotatably mounted inside the housing and extends out of the housing. Then, the first knob is sleeved over the portion of the first cable pulley that extends out of the housing. The first matching portion and the third matching portion are matched, and the first latching portion and the third latching portion are latch-fitted, such that the first cable pulley and the first knob are securely connected, ensuring that the first cable pulley is stably mounted in the housing. In addition, with the first matching portion and the third matching portion, the first knob can drive the first cable pulley to rotate, so as to stably control the bending of the bending section. Similarly, the second cable pulley is rotatably mounted inside the housing, passes through the first perforation, and extends out of the housing. Then, the second knob is sleeved over the portion of the second cable pulley that extends out of the housing. The second matching portion and the fourth matching portion are matched, and the second latching portion and the fourth latching are latch-fitted, such that the second cable pulley and the second knob are securely connected, ensuring that the second cable pulley is stably mounted in the housing. In addition, with the second matching portion and the fourth matching portion, the second knob can drive the second cable pulley to rotate, so as to stably control the bending of the bending section. The whole structure is ingeniously designed, the first knob and the second knob only need to be sleeved to complete the installation, which is convenient for the assembly of the product, improving the assembly efficiency, thereby effectively reducing the production cost.

### BRIEF DESCRIPTION OF THE DRAWINGS

The accompanying drawings forming a part of the disclosure are used to provide a further understanding of the present disclosure. The illustrative embodiments of the present disclosure and descriptions thereof are used to explain the present disclosure and do not constitute an improper limitation of the present disclosure.

In order to illustrate the technical solutions of the embodiments of the present application more clear, the accompanying drawings used in the description of the embodiments will be briefly introduced below. It is apparent that the accompanying drawings described herein are only some embodiments of the present application. For those of ordinary skill in the art, other drawings can also be obtained from these drawings without creative efforts.
FIG. 1 is a schematic diagram showing a structure of an endoscope handle according to an embodiment.
FIG. 2 is a cross-sectional view showing a structure of an endoscope handle according to an embodiment.
FIG. 3 is an enlarged schematic view of the structure at circle A in FIG. 2;
FIG. 4 is an exploded schematic view showing a structure of an endoscope handle according to an embodiment.
FIG. 5 is a schematic structural diagram of a first cable pulley according to an embodiment.
FIG. 6 is a first schematic structural diagram of a first knob according to an embodiment.
FIG. 7 is a second schematic structural diagram of the first knob according to an embodiment.
FIG. 8 is a first schematic structural diagram of a first bracket according to an embodiment.
FIG. 9 is a second schematic structural diagram of the first bracket according to an embodiment.
FIG. 10 is a first schematic structural diagram of a second cable pulley according to an embodiment;
FIG. 11 is a second schematic structural diagram of the second cable pulley according to an embodiment.
FIG. 12 is a first schematic structural diagram of a second knob according to an embodiment.
FIG. 13 is a second schematic structural diagram of the second knob according to an embodiment.

100. Endoscope knob device; 110. Housing; 111. mounting hole; 120. First cable pulley; 121. First perforation; 122. First matching portion; 1221. First matching groove; 123. First latching portion; 1231. First latching block; 1232. First alignment portion; 124. First winding portion; 125. First mounting portion; 126. Third limiting portion; 127. Second sealing ring; 130. Second cable pulley; 131. Second perforation; 132. Second matching portion; 1321, Second matching groove; 133. Second latching portion; 1331. Second latching groove; 1332. Third alignment portion; 134. Second winding portion; 135. Second mounting portion; 136. Fourth limiting portion; 140. First knob; 141. Third matching portion; 1411. First matching block; 142. Third latching portion; 1421. First latching groove; 1422. Second alignment portion; 143. First protrusion; 144. First through hole; 150. Second knob; 151. Fourth matching portion; 1511. Second matching block; 152. Fourth latching portion; 1521. Second latching block; 1522. Fourth alignment portion; 153. Mounting groove; 154. Second protrusion; 155. Brake ring; 156. Second through hole; 160. First bracket; 161 . First limiting portion; 162. Second limiting portion; 163. First sealing ring; 170. Second bracket; 200. First braking assembly; 300. Second braking assembly.

### DETAILED DESCRIPTION OF THE EMBODIMENTS

In order to make the above purposes, features and advantages of the present disclosure more obvious and understandable, specific embodiments of the present disclosure will be described in detail below with reference to the accompanying drawings. Some specific details are set forth in the following description in order to facilitate a thorough understanding of the present disclosure. However, the present disclosure can be implemented in many other ways different from those described herein. Those skilled in the art can make similar improvements without departing from the connotation of the present disclosure. Therefore, the present disclosure is not limited to the specific embodiments disclosed below.

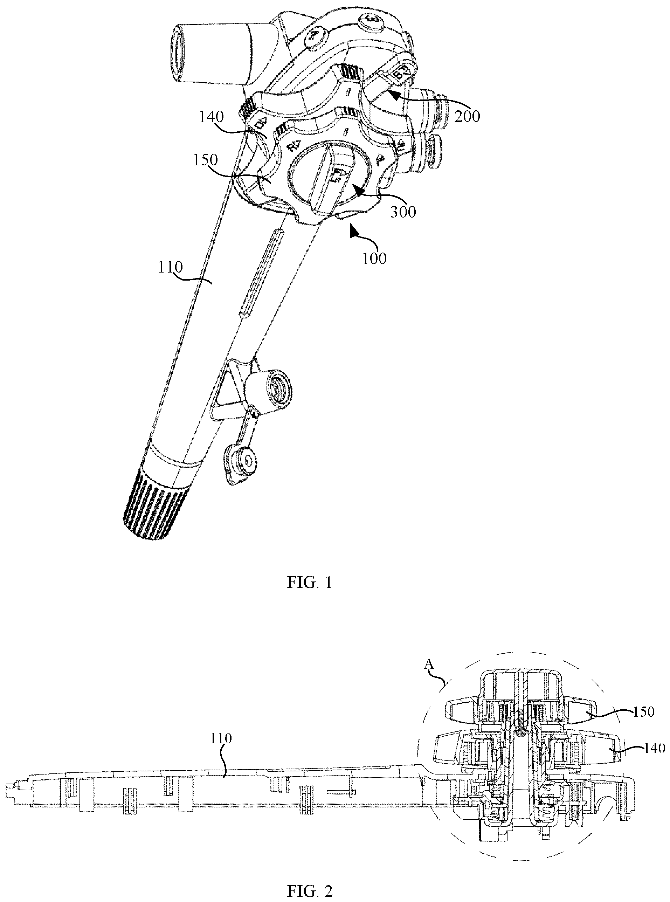

In an embodiment, referring to FIG. 1, FIG. 2, FIG. 3, and FIG. 4, an endoscope knob device 100 is provided. The endoscope knob device 100 includes a housing 110, a first cable pulley 120, a second cable pulley 130, a first knob 140, and a second knob 150. The housing 110 includes a mounting hole 111 that communicates the inside and outside of the housing 110. A first perforation 121 is defined throughout the first cable pulley 120. The first cable pulley 120 is rotatably mounted in the housing 110 and extends out of the housing 110 through the mounting hole 111. The first cable pulley 120 includes a first matching portion 122 and a first latching portion 123, which are located outside the housing 110. The second cable pulley 130 is rotatably mounted in the housing 110 and extends out of the housing 110 through the first perforation 121. The second cable pulley 130 includes a second matching portion 132 and a second latching portion 133, which are located outside the housing 110. The first knob 140 and the second knob 150 are both located outside the housing 110 and are sleeved over the first cable pulley 120 and the second cable pulley 130, respectively. The first knob 140 includes a third matching portion 141 and a third latching portion 142 that is latch-fitted with the first latching portion 123. The third matching portion 141 cooperates with the first matching portion 122 to enable the first knob 140 to drive the first cable pulley 120 to rotate. The second knob 150 includes a fourth matching portion 151 and a fourth latching portion 152 that is latch-fitted with the second latching portion 133. The fourth matching portion 151 cooperates with the second matching portion 132 to enable the second knob 150 to drive the second cable pulley 130 to rotate.

According to the above endoscope knob device 100, during the assembly process, the first cable pulley 120 is rotatably mounted inside the housing 110 and extends out of the housing 110. Then, the first knob 140 is sleeved over the portion of the first cable pulley 120 that extends out of the housing 110. The first matching portion 122 and the third matching portion 141 are matched, and the first latching portion 123 and the third latching portion 142 are latch-fitted, such that the first cable pulley 120 and the first knob 140 are securely connected, ensuring that the first cable pulley 120 is stably mounted in the housing 110. In addition, with the first matching portion 122 and the third matching portion 141, the first knob 140 can drive the first cable pulley 120 to rotate, so as to stably control the bending of the bending section. Similarly, the second cable pulley 130 is rotatably mounted inside the housing 110, passes through the first perforation 121, and extends out of the housing 110. Then, the second knob 150 is sleeved over the portion of the second cable pulley 130 that extends out of the housing 110. The second matching portion 132 and the fourth matching portion 151 are matched, and the second latching portion 133 and the fourth latching 152 are latch-fitted, such that the second cable pulley 130 and the second knob 150 are securely connected, ensuring that the second cable pulley 130 is stably mounted in the housing 110. In addition, with the second matching portion 132 and the fourth matching portion 151, the second knob 150 can drive the second cable pulley 130 to rotate, so as to stably control the bending of the bending section. The whole structure is ingeniously designed, the first knob 140 and the second knob 150 only need to be sleeved to complete the installation, which is convenient for the assembly of the product, improving the assembly efficiency, thereby effectively reducing the production cost.

It should be noted that the first matching portion 122 and the third matching portion 141 can limit the relative rotation between the first cable pulley 120 and the first knob 140, so that the first knob 140 can drive the first cable pulley 120 to rotate with it. There are various designs for the structure of the first matching portion 122 and the third matching portion 141. For example, the first matching portion 122 is a convex structure, and the second matching portion 132 is a groove or a hole. Alternatively, the first matching portion 122 is a groove or a hole, and the second matching portion 132 is a convex structure, etc.

Similarly, the second matching portion 132 and the fourth matching portion 151 can limit the relative rotation between the second cable pulley 130 and the second knob 150, so that the second knob 150 can drive the second cable pulley 130 to rotate with it. There are various designs for the structure of the second matching portion 132 and the fourth matching portion 151. For example, the second matching portion 132 is a convex structure, and the fourth matching portion 151 is a groove or a hole. Alternatively, the second matching portion 132 is a groove or a hole, and the fourth matching portion 151 is a convex structure, etc.

It should also be noted that the movement of the first cable pulley 120 is limited by the cooperation between the first latching portion 123 and the third latching portion 142, preventing the first cable pulley 120 from falling off from the mounting hole 111 of the housing 110, thereby ensuring that the first cable pulley 120 is stably mounted in the housing 110. In addition, the movement of the second cable pulley 130 is limited by the cooperation between the second latching portion 133 and the fourth latching portion 152, preventing the second cable pulley 130 from falling off from the mounting hole 111 of the housing 110, thereby ensuring that the second cable pulley 130 is stably mounted in the housing 110.

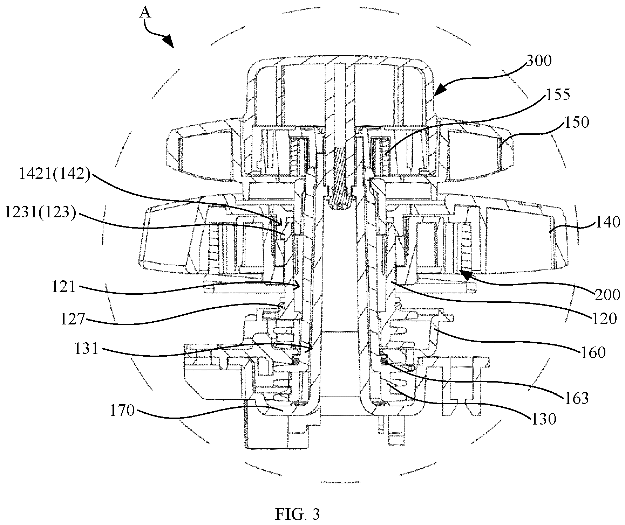

Further, referring to FIG. 3, the endoscope knob device 100 further includes a first bracket 160 mounted inside the housing 110. The first bracket 160 is sleeved on the second cable pulley 130 and covers the first cable pulley 120. In this way, the first cable pulley 120 is stably mounted in the housing 110 through the first bracket 160. Moreover, the first bracket 160 can provide protection for the first cable pulley 120 so that the traction wire can be wound around the first cable pulley 120.

Optionally, the first bracket 160 may be connected to the housing 110 through, but is not limited to, a bolt connection, clamping connection, riveting, welding, bonding, etc.

Further, referring to FIG. 3, the endoscope knob device 100 further includes a first sealing ring 163, which is arranged between the first bracket 160 and the second cable pulley 130, improving the sealing performance between the first bracket 160 and the second cable pulley 130 and avoiding corrosion and damage to the first cable pulley 120 caused by the infiltration of liquid from the first bracket 160 to the second cable pulley 130.

It should be understood that the endoscope knob device 100 also includes a second sealing ring 127, which is arranged between the first cable pulley 120 and the housing 110, further improving the sealing performance of the endoscope knob device 100 and effectively preventing liquid from entering the first cable pulley 120 through the mounting hole 111 from outside of the housing 110.

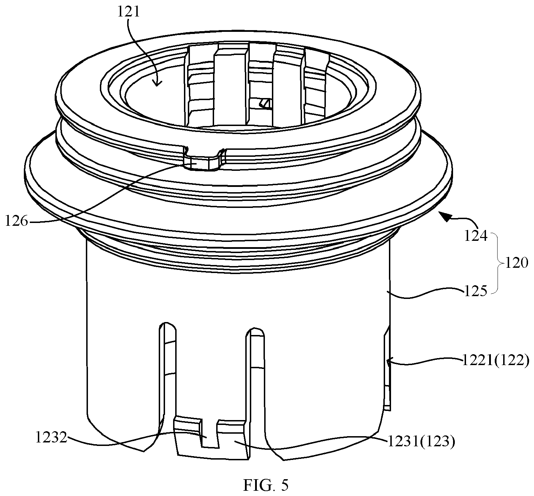

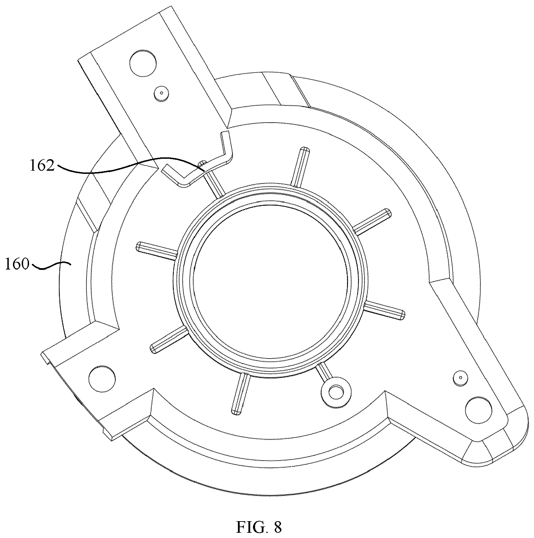

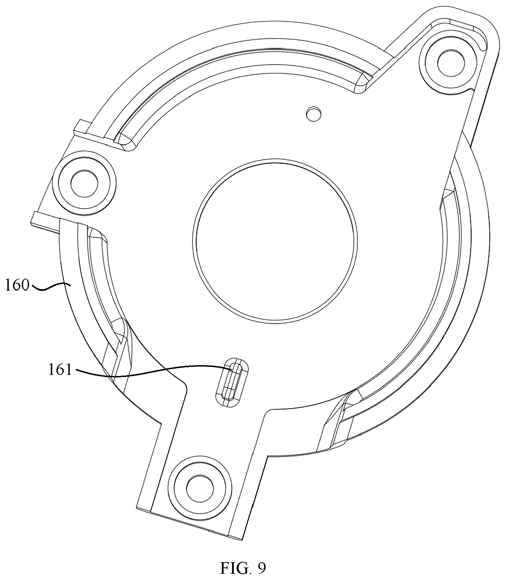

In an embodiment, referring to FIG. 5, FIG. 8, and FIG. 9, a first limiting portion 161 and a second limiting part 162 are disposed at opposite ends of the first bracket 160, respectively. Athird limiting portion 126 is disposed on the first cable pulley 120. The third limiting portion 126 cooperates with the first limiting portion 161 to limit the rotation angle of the first cable pulley 120. A fourth limiting portion 136 is disposed on the second cable pulley 130. The fourth limiting portion 136 cooperates with the second limiting portion 162 to limit the rotation angle of the second cable pulley 130. It can be seen that when the first knob 140 drives the first cable pulley 120 to rotate to a preset angle, the third limiting portion 126 cooperates with the first limiting portion 161 to limit the further rotation of the first cable pulley 120, preventing the traction wire from breaking due to excessive rotation of the first cable pulley 120, thereby ensuring the safety of the endoscope. Similarly, when the second knob 150 drives the second cable pulley 130 to rotate to a preset angle, the fourth limiting portion 136 cooperates with the second limiting portion 162 to limit the further rotation of the second cable pulley, preventing the traction wire from breaking due to excessive rotation of the second cable pulley 130, thereby ensuring the safety of the endoscope.

It should be noted that the first limiting portion 161, the second limiting portion 162, the third limiting portion 126, and the fourth limiting portion 136 all have a convex structure. The rotation ranges of the first cable pulley 120 and the second cable pulley 130 are effectively controlled by utilizing the contact interaction between the convex structures.

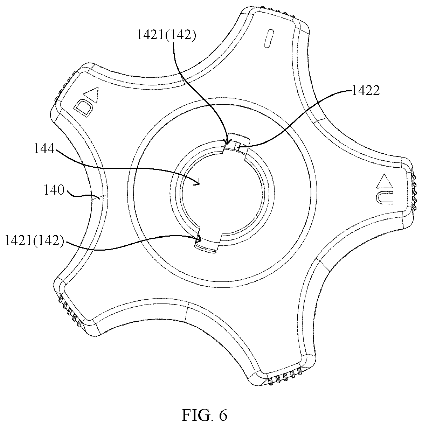

Further, referring to FIG. 5 and FIG. 6, there are at least two first latching portions 123 and at least two third latching portions 142. The at least two first latching portions 123 are arranged at intervals along the circumferential direction of the first cable pulley 120. The at least two third latching portions 142 and the at least two first latching portions 123 are arranged in one-to-one correspondence. In this way, the first cable pulley 120 and the first knob 140 are effectively engaged through the one-to-one cooperation of the first latching portions 123 and the third latching portions 142, so that the connection between the first cable pulley 120 and the first knob 140 is more secure and stable.

In an embodiment, referring to FIG. 5 and FIG. 6, each first latching portion 123 includes a first latching block 1231. Each third latching portion 142 includes a first latching groove 1421 that matches with the first latching block 1231. In this way, the first cable pulley 120 and the first knob 140 are stably connected through the cooperation between the first latching block 1231 and the first latching groove 1421.

In addition, one of the first latching blocks 1231 includes a first alignment portion 1232. One of the first latching grooves 1421 includes a second alignment portion 1422, which matches with the first alignment portion 1232, on the groove wall. Therefore, during assembly, the first knob 140 can be stably latched onto the first cable pulley 120 by simply aligning the first alignment portion 1232 with the second alignment portion 1422, making the assembly of the endoscope knob device 100 more convenient, thereby improving the assembly efficiency of the endoscope.

It should be noted that the first alignment portion 1232 and the second alignment portion 1422 have various forms. For example, the first alignment portion 1232 is designed as a convex block, and the second alignment portion 1422 is designed as a groove. Alternatively, both the first alignment portion 1232 and the second alignment portion 1422 are designed with corresponding patterns, markings, etc.

Specifically, the first alignment portion 1232 is a groove, and the second alignment portion 1422 is a convex block.

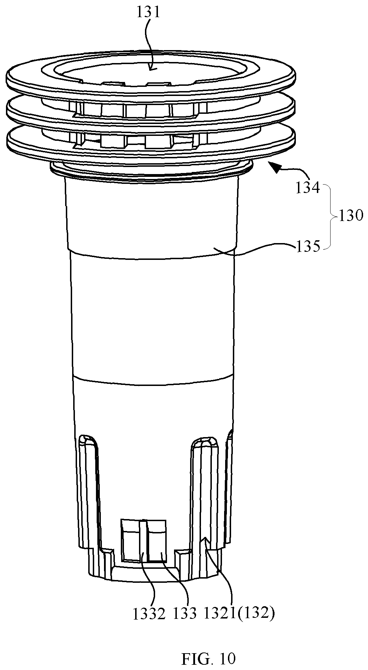

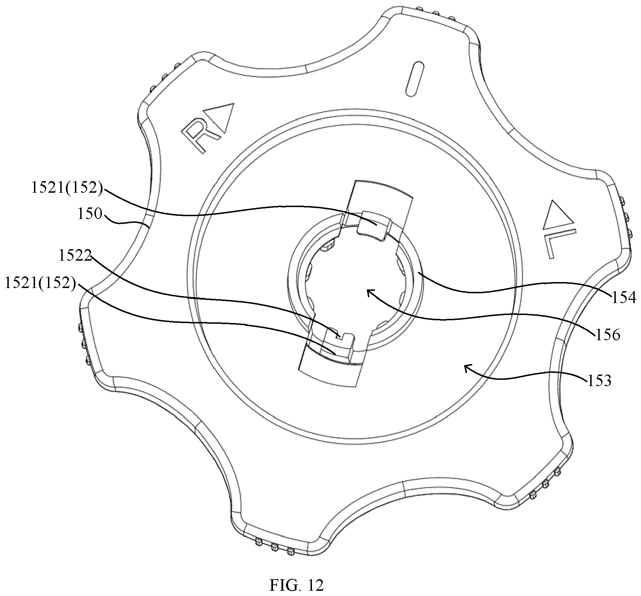

Further, referring to FIG. 10 and FIG. 12, there are at least two second latching portions 133 and at least two fourth latching portions 152. The at least two second latching portions 133 are arranged at intervals along the circumferential direction of the second cable pulley 130. The at least two fourth latching portions 152 and the at least two second latching portions 133 are arranged in one-to-one correspondence. In this way, the second cable pulley 130 and the second knob 150 are effectively latched through the one-to-one cooperation of the second latching portions 133 and the fourth latching portions 152, so that the connection between the second cable pulley 130 and the second knob 150 is more secure and stable.

In an embodiment, referring to FIG. 10 and FIG. 12, each second latching portion 133 includes a second latching groove 1331. Each fourth latching portion 152 includes a second latching block 1521 that matches with the second latching groove 1331. In this way, the second cable pulley 130 and the second knob 150 are stably connected through the cooperation between the second latching blocks 1521 and the second latching grooves 1331.

In addition, the wall of one of the second latching grooves 1331 defines a third alignment portion 1332. One of the second latching blocks 1521 includes a fourth alignment portion 1522 that matches with the third alignment portion 1332. Therefore, during assembly, the second knob 150 can be stably latched onto the second cable pulley 130 by simply aligning the third alignment portion 1332 with the fourth alignment portion 1522, making the assembly of the endoscope knob device 100 more convenient, thereby improving the assembly efficiency of the endoscope.

It should be noted that the third alignment portion 1332 and the fourth alignment portion 1522 have various forms. For example, the third alignment portion 1332 is designed as a groove, and the fourth alignment portion 1522 is designed as a convex block. Alternatively, both the third alignment portion 1332 and the fourth alignment portion 1522 are designed with corresponding patterns, markings, etc.

Specifically, the third alignment portion 1332 is a convex block, and the fourth alignment portion 1522 is a groove.

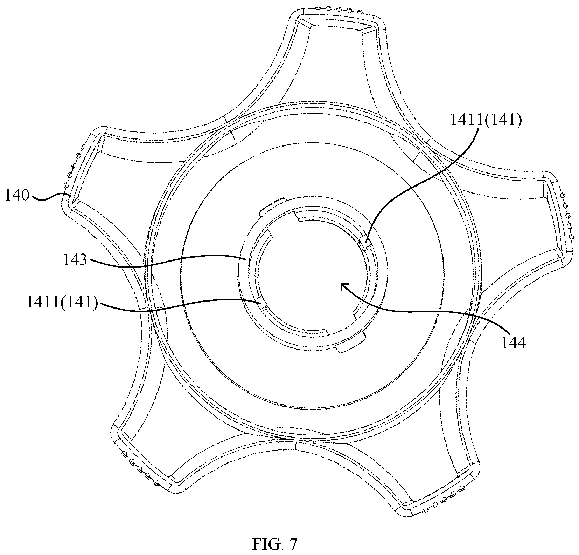

Further, referring to FIG. 5 and FIG. 7, there are at least two first matching portions 122 and at least two third matching portions 141. The at least two first matching portions 122 are arranged at intervals along the circumferential direction of the first cable pulley 120. The at least two third matching portions 141 and the at least two first matching portions 122 are arranged in one-to-one correspondence. In this way, the first cable pulley 120 and the first knob 140 are effectively cooperated through the one-to-one cooperation between the first matching portions 122 and the third matching portions 141, so that the first knob 140 drives the first cable pulley 120 to rotate more securely and stably.

In an embodiment, referring to FIG. 5 and FIG. 7, each first matching portion 122 includes a first matching groove 1221. Each third matching portion 141 includes a first matching block 1411 that matches with the first matching groove 1221. Each first matching groove 1221 extends along the axial direction of the first cable pulley 120. In this way, the first matching blocks 1411 are inserted into the first matching grooves 1221, respectively, so that the first cable pulley 120 cannot rotate with respect to the first knob 140, and the first knob 140 can drive the first cable pulley 120 to rotate with it.

Specifically, referring to FIG. 5 and FIG.7, the first matching groove 1221 extends along the axial direction of the first cable pulley 120, starting from one end of the first cable pulley 120. In this way, the first matching block 1411 can be inserted into the first matching groove 1221 more conveniently, making the connection between the first cable pulley 120 and the first knob 140 more convenient.

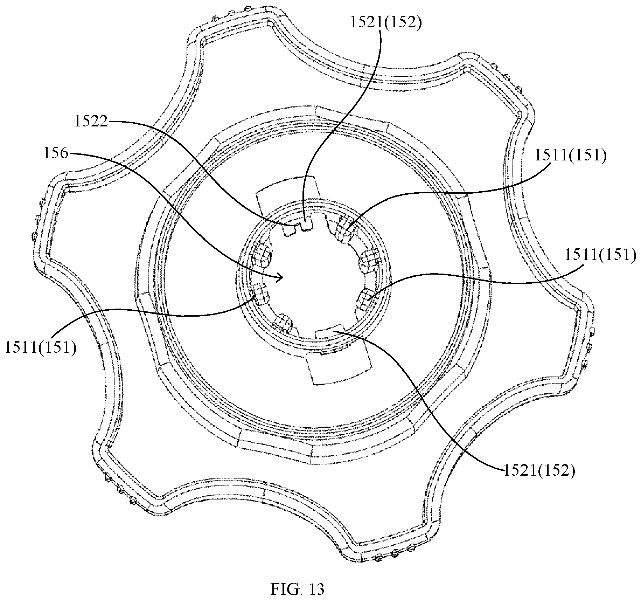

Further, referring to FIG. 10 and FIG. 13, there are at least two second matching portions 132 and at least two fourth matching portions 151. The at least two second matching portions 132 are arranged at intervals along the circumferential direction of the second cable pulley 130. The at least two second matching portions 132 and the at least two fourth matching portions 151 are arranged in one-to-one correspondence. In this way, the second cable pulley 130 and the second knob 150 are effectively engaged through the one-to-one cooperation between the second matching portions 132 and the fourth matching portions 151, so that the second knob 150 drives the second cable pulley 130 to rotate more securely and stably.

In an embodiment, referring to FIG. 10 and FIG. 13, each second matching portion 132 includes a second matching groove 1321. Each fourth matching portion 151 includes a second matching block 1511 that matches with the second matching groove 1321. Each second matching groove 1321 extends along the axial direction of the second cable pulley 130. In this way, the second matching blocks 1511 are inserted into the second matching grooves 1321, respectively, so that the second cable pulley 130 cannot rotate with respect to the second knob 150, and the second knob 150 can drive the second cable pulley 130 to rotate with it.

Specifically, referring to FIG. 10 and FIG. 13, the second matching groove 1321 extends along the axial direction of the second cable pulley 130, starting from one end of the second cable pulley 130. In this way, the second matching block 1511 can be inserted into the second matching groove 1321 more conveniently, making the connection between the second cable pulley 130 and the second knob 150 more convenient.

In an embodiment, referring to FIG. 3, FIG. 6, and FIG. 7, the first knob 140 defines a first through hole 144 for the first cable pulley 120 to pass through. The at least two third matching portions 141 are arranged at intervals and surround the first through hole 144. The at least two third latching portions 142 are arranged at intervals and surround the first through hole 144. When the first cable pulley 120 is inserted into the first through hole 144, the first matching portions 122 are fitted on the third matching portions 141, respectively, and the first latching portions 123 are latched with the third latching portions 142, respectively. In this way, during assembly, the second cable pulley 130 and the second knob 150 can be stably engaged by simply inserting the first cable pulley 120 into the first through hole 144.

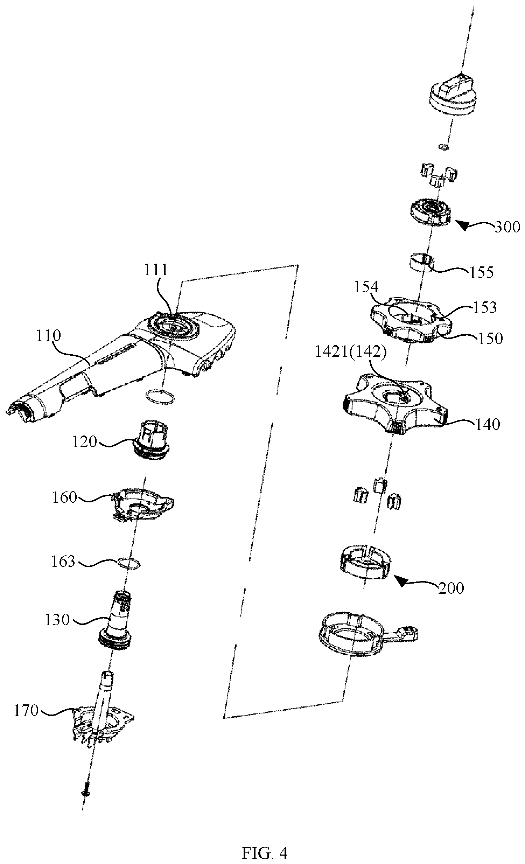

In an embodiment, referring to FIG. 4 and FIG. 7, a first protrusion 143 is disposed on the side of the first knob 140 facing the housing 110, surrounding the first through hole 144. The first protrusion 143 is configured to cooperate with a first braking assembly 200 for braking. In this way, the first knob 140 is stably braked by the first braking assembly 200, so that the bending angle of the bending section is fixed, facilitating the operator to observe images at a specific angle.

In an embodiment, referring to FIG. 3, FIG. 12, and FIG. 13, the second knob 150 defines a second through hole 156 for the second cable pulley 130 to pass through. The at least two fourth matching portions 151 are arranged at intervals and surround the second through hole 156. The at least two fourth latching portions 152 are arranged at intervals and surround the second through hole 156. When the second cable pulley 130 is inserted into the second through hole 156, the second matching portions 132 are fitted on the fourth matching portions 151, respectively, and the second latching portions 133 are latched with the fourth latching portions 152, respectively. In this way, during assembly, a stable engagement between the second cable pulley 130 and the second knob 150 can be achieved by simply inserting the second cable pulley 130 into the second through hole 156.

In an embodiment, referring to FIG. 4 and FIG. 12, a mounting groove 153 for the second braking assembly 300 to be mounted is defined on the side of the second knob 150 away from the first knob 140. A second protrusion 154 is disposed on the wall of the mounting groove 153, surrounding the second through hole 156. The second protrusion 154 is configured to cooperate with a second braking assembly 300 to provide braking. In this way, the second knob 150 is stably braked by the second braking assembly 300, so that the bending angle of the bending section is fixed, facilitating the operator to observe images at a specific angle.

In an embodiment, referring to FIG. 3 and FIG. 4, the endoscope knob device 100 further includes a braking ring 155, which is sleeved on the second protrusion 154. The second protrusion 154 cooperates with the second braking assembly 300 through the braking ring 155, achieving a better braking effect.

In an embodiment, referring to FIG. 3 and FIG. 4, the endoscope knob device 100 further includes a second bracket 170 mounted in the housing 110. A second perforation 131 is defined in the second cable pulley 130. The second bracket 170 covers the second cable pulley 130 and extends out of the housing 110 through the second perforation 131 to connect to the second braking assembly 300. Such a design makes the endoscope handle structure more compact and reasonable, making the operation of the endoscope more stable.

In an embodiment, referring to FIG. 5, the first cable pulley 120 includes a first winding portion 124 and a first mounting portion 125 that is disposed on the first winding portion 124. The first perforation 121 extends throughout the first winding portion 124 and the first mounting portion 125. The first winding portion 124 is located in the housing 110. The first mounting portion 125 passes through the mounting hole 111. The first matching portions 122 and the first latching portions 123 are arranged on the first mounting portion 125. In this way, the first cable pulley 120 can be mounted in the housing 110 more stably.

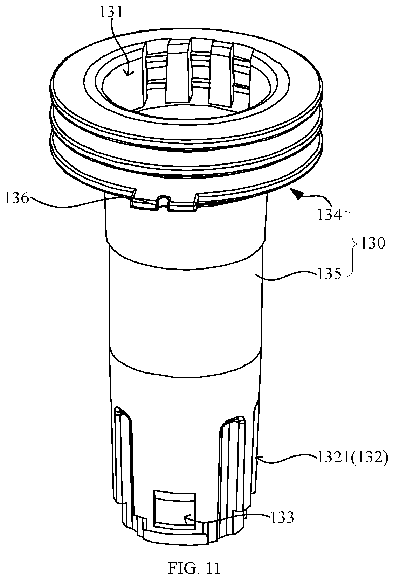

In an embodiment, referring to FIG. 10 and FIG. 11, the second cable pulley 130 includes a second winding portion 134 and a second mounting portion 135 that is disposed on the second winding portion 134. The second winding portion 134 is located in the housing 110. The second mounting portion 135 passes through the first perforation 121. The second matching portions 132 and the second latching portions 133 are located on the second mounting portion 135. In this way, the second cable pulley 130 can be mounted in the housing 110 more stably.

In an embodiment, referring to FIG. 1, an endoscope handle is provided. The endoscope handle includes a first braking assembly 200, a second braking assembly 300, and an endoscope knob device 100 in any of the above embodiments. The first braking assembly 200 is configured to brake the first knob 140 or the first cable pulley 120, and the second braking assembly 300 is configured to brake the second knob 150 or the second cable pulley 130.

The above-mentioned endoscope handle employs the above endoscope knob device 100. During assembly, the first cable pulley 120 is rotatably mounted inside the housing 110 and extends out of the housing 110. Then, the first knob 140 is sleeved over the portion of the first cable pulley 120 that extends out of the housing 110. The first matching portion 122 and the third matching portion 141 are matched, and the first latching portion 123 and the third latching portion 142 are latch-fitted, such that the first cable pulley 120 and the first knob 140 are securely connected, ensuring that the first cable pulley 120 is stably mounted in the housing 110. In addition, with the first matching portion 122 and the third matching portion 141, the first knob 140 can drive the first cable pulley 120 to rotate, so as to stably control the bending of the bending section. Similarly, the second cable pulley 130 is rotatably mounted inside the housing 110, passes through the first perforation 121, and extends out of the housing 110. Then, the second knob 150 is sleeved over the portion of the second cable pulley 130 that extends out of the housing 110. The second matching portion 132 and the fourth matching portion 151 are matched, and the second latching portion 133 and the fourth latching 152 are latch-fitted, such that the second cable pulley 130 and the second knob 150 are securely connected, ensuring that the second cable pulley 130 is stably mounted in the housing 110. In addition, with the second matching portion 132 and the fourth matching portion 151, the second knob 150 can drive the second cable pulley 130 to rotate, so as to stably control the bending of the bending section. The whole structure is ingeniously designed, the first knob 140 and the second knob 150 only need to be sleeved to complete the installation, which is convenient for the assembly of the product, improving the assembly efficiency, thereby effectively reducing the production cost.

In an embodiment, referring to FIG. 1, an endoscope is provided. The endoscope includes the endoscope handle in the above embodiments.

The above-mentioned endoscope employs the above endoscope handle. During assembly, the first cable pulley 120 is rotatably mounted inside the housing 110 and extends out of the housing 110. Then, the first knob 140 is sleeved over the portion of the first cable pulley 120 that extends out of the housing 110. The first matching portion 122 and the third matching portion 141 are matched, and the first latching portion 123 and the third latching portion 142 are latch-fitted, such that the first cable pulley 120 and the first knob 140 are securely connected, ensuring that the first cable pulley 120 is stably mounted in the housing 110. In addition, with the first matching portion 122 and the third matching portion 141, the first knob 140 can drive the first cable pulley 120 to rotate, so as to stably control the bending of the bending section. Similarly, the second cable pulley 130 is rotatably mounted inside the housing 110, passes through the first perforation 121, and extends out of the housing 110. Then, the second knob 150 is sleeved over the portion of the second cable pulley 130 that extends out of the housing 110. The second matching portion 132 and the fourth matching portion 151 are matched, and the second latching portion 133 and the fourth latching 152 are latch-fitted, such that the second cable pulley 130 and the second knob 150 are securely connected, ensuring that the second cable pulley 130 is stably mounted in the housing 110. In addition, with the second matching portion 132 and the fourth matching portion 151, the second knob 150 can drive the second cable pulley 130 to rotate, so as to stably control the bending of the bending section. The whole structure is ingeniously designed, the first knob 140 and the second knob 150 only need to be sleeved to complete the installation, which is convenient for the assembly of the product, improving the assembly efficiency, thereby effectively reducing the production cost.

The technical features in the above embodiments may be randomly combined. For brevity, not all possible combinations of the technical features in the above embodiments are described. However, all the combinations of the technical features should be considered to be included within the scope of the present disclosure, as long as the combinations are not contradictory.

The above-mentioned embodiments only illustrate several embodiments of the present disclosure, and the descriptions of which are relatively specific and detailed, but should not be construed as limitations to the scope of the present disclosure. It should be noted that, for those skilled in the art, variations and improvements can be made without departing from the concept of the present disclosure, which all belong to the protection scope of the present disclosure. Therefore, the protection scope of the present disclosure shall be subject to the appended claims.

In the description of the present disclosure, it should be understood that the orientation or positional relationships indicated by the terms "center", "longitudinal", "transverse", "length", "width", "thickness", "above", "below", "front", "back", "left", "right", "vertical", "horizontal", "top", "bottom", "inside", "outside", "clockwise", "counterclockwise", "axial", "radial direction", "circumferential direction", etc. are based on the orientation or positional relationships shown in the figures, and are merely for the purpose of facilitating the description of the present disclosure and simplifying the description, and do not indicate or imply the device or component referred to must have a specific orientation or must be constructed and operate in a specific orientation, and therefore should not be construed as a limitation of the present disclosure.

In addition, the terms "first" and "second" are used merely for the purpose of description, and should not be construed as indicating or implying relative importance or implicitly indicating a quantity of indicated technical features. Therefore, features defined by "first" or "second" may explicitly or implicitly include at least one of such features. In description of this application, "a plurality of" means at least two, such as two or three unless it is specifically defined otherwise.

In the present disclosure, unless otherwise clearly stated and limited, the terms "installed", "connected", "communicated", "fixed", etc. should be interpreted broadly. For example, it may be a fixed connection, a detachable connection, or integrated. It may also be a mechanical connection or an electrical connection. It may be a direct connection or an indirect connection through an intermediate medium. It may also be an internal connection between two elements or an interactive relationship between two elements, unless otherwise specified limitations. For those of ordinary skill in the art, the specific meanings of the above terms in the present disclosure can be understood according to specific situations.

In the present disclosure, unless otherwise expressly stated and limited, a first feature being "on" or "below" a second feature may refer to a direct connection between the first feature and the second feature or an indirect connection between the first feature and the second feature through an intermediate medium. Furthermore, the first feature being "on", "above" and "over" the second feature may be that the first feature is directly above or diagonally above the second feature, or simply that the first feature is horizontally higher than the second feature. The first feature being "under", "below" and "beneath" the second feature may be that the first feature is directly below or diagonally below the second feature, or simply that the first feature has a smaller horizontal height than the second feature.

It should be noted that when an element is referred to as being "mounted" or "disposed on" another element, it may be directly on the other element or connected to the other element through intervening elements. When an element is referred to as being "connected" to another element, it may be directly connected to the other element or there may also be intervening elements. The terms "vertical", "horizontal", "above", "below", "left", "right", and similar expressions used herein are merely for illustrative purposes and do not imply the only implementation manner.

## Claims

1. An endoscope knob device, comprising:
a housing defining a mounting hole that communicates inside and outside of the housing;
a first cable pulley defining a first perforation, the first cable pulley being rotatably mounted in the housing and extending out of the housing through the mounting hole, the first cable pulley comprising a first matching portion and a first latching portion, which are located outside the housing;
a second cable pulley rotatably mounted in the housing and extending out of the housing through the first perforation, the second cable pulley comprising a second matching portion and a second latching portion, which are located outside the housing; and
a first knob and a second knob that are both located outside the housing, wherein the first knob and the second knob are sleeved over the first cable pulley and the second cable pulley, respectively, the first knob comprises a third matching portion and a third latching portion latch-fitted with the first latching portion, the third matching portion cooperates with the first matching portion, so that the first knob drives the first cable pulley to rotate, the second knob comprises a fourth matching portion and a fourth latching portion latch-fitted with the second latching portion, and the fourth matching portion cooperates with the second matching portion, so that the second knob drives the second cable pulley to rotate.

2. The endoscope knob device according to claim 1, wherein the endoscope knob device further comprises a first bracket mounted in the housing, and the first bracket is sleeved on the second cable pulley and covers the first cable pulley.

3. The endoscope knob device according to claim 2, wherein the endoscope knob device further comprises a first sealing ring arranged between the first bracket and the second cable pulley.

4. The endoscope knob device according to claim 1, wherein the endoscope knob device further comprises a second sealing ring arranged between the first cable pulley and the housing.

5. The endoscope knob device according to claim 2, wherein a first limiting portion and a second limiting portion are disposed at opposite ends of the first bracket, respectively, the first cable pulley comprises a third limiting portion that cooperates with the first limiting portion to limit a rotation angle of the first cable pulley, and the second cable pulley comprises a fourth limiting portion that cooperates with the second limiting portion to limit a rotation angle of the second cable pulley.

6. The endoscope knob device according to claim 1, wherein at least two first latching portions and at least two third latching portions are configured, the at least two first latching portions are arranged at intervals along a circumferential direction of the first cable pulley, and the at least two third latching portions and the at least two first latching portions are arranged in one-to-one correspondence.

7. The endoscope knob device according to claim 6, wherein each first latching portion comprises a first latching block, each third latching portion comprises a first latching groove that matches with the first latching block, one of the first latching blocks comprises a first alignment portion, and a wall of one of the first latching grooves comprises a second alignment portion that matches with the first alignment portion.

8. The endoscope knob device according to claim 1, wherein at least two second latching portions and at least two fourth latching portions are configured, the at least two second latching portions are arranged at intervals along a circumferential direction of the second cable pulley, and the at least two fourth latching portions and the at least two second latching portions are arranged in one-to-one correspondence.

9. The endoscope knob device according to claim 8, wherein each second latching portion comprises a second latching groove, each fourth latching portion comprises a second latching block that matches with the second latching groove, a wall of one of the second latching grooves comprises a third alignment portion, and one of the second latching blocks comprises a fourth alignment portion that matches with the third alignment portion.

10. The endoscope knob device according to claim 1, wherein at least two first matching portions and at least two third matching portions are configured, the at least two first matching portions are arranged at intervals along a circumferential direction of the first cable pulley, and the at least two third matching portions and the at least two first matching portions are arranged in one-to-one correspondence.

11. The endoscope knob device according to claim 10, wherein each first matching portion comprises a first matching groove, each third matching portion comprises a first matching block that matches with the first matching groove, and the first matching groove extends along an axial direction of the first cable pulley.

12. The endoscope knob device according to claim 1, wherein at least two second matching portions and at least two fourth matching portions are configured, the at least two second matching portions are arranged at intervals along a circumferential direction of the second cable pulley, and the at least two second matching portions and the at least two fourth matching portions are arranged in one-to-one correspondence.

13. The endoscope knob device according to claim 12, wherein each second matching portion comprises a second matching groove, each fourth matching portion comprises a second matching block that matches with the second matching groove, and the second matching groove extends along an axial direction of the second cable pulley.

14. The endoscope knob device according to claim 1, wherein at least two first latching portions and at least two third latching portions are configured, the at least two first latching portions are arranged at intervals along a circumferential direction of the first cable pulley, and the at least two third latching portions and the at least two first latching portions are arranged in one-to-one correspondence;
at least two first matching portions and at least two third matching portions are configured, the at least two first matching portions are arranged at intervals along a circumferential direction of the first cable pulley, and the at least two third matching portions and the at least two first matching portions are arranged in one-to-one correspondence;
the first knob defines a first through hole for the first cable pulley to pass through, the at least two third matching portions are arranged at intervals and surround the first through hole, and the at least two third latching portions are arranged at intervals and surround the first through hole.

15. The endoscope knob device according to claim 14, wherein a first protrusion is disposed on a side of the first knob facing the housing, surrounding the first through hole, and the first protrusion is configured to cooperate with a first braking assembly for braking.

16. The endoscope knob device according to claim 1, wherein at least two second latching portions and at least two fourth latching portions are configured, the at least two second latching portions are arranged at intervals along a circumferential direction of the second cable pulley, and the at least two fourth latching portions and the at least two second latching portions are arranged in one-to-one correspondence;
at least two second matching portions and at least two fourth matching portions are configured, the at least two second matching portions are arranged at intervals along a circumferential direction of the second cable pulley, and the at least two second matching portions and the at least two fourth matching portions are arranged in one-to-one correspondence; and
the second knob defines a second through hole for the second cable pulley to pass through, the at least two fourth matching portions are arranged at intervals and surround the second through hole, and the at least two fourth latching portions are arranged at intervals and surround the second through hole.

17. The endoscope knob device according to claim 16, wherein a mounting groove for a second braking assembly to be mounted is defined on a side of the second knob away from the first knob, a second protrusion is disposed on a wall of the mounting groove, surrounding the second through hole, and the second protrusion is configured to cooperate with the second braking assembly for braking.

18. The endoscope knob device according to claim 1, wherein the endoscope knob device further comprises a second bracket mounted in the housing, the second cable pulley defines a second perforation, and the second bracket covers the second cable pulley and extends out of the housing through the second perforation to connect to a second braking assembly.

19. The endoscope knob device according to claims 1 to 18, wherein the first cable pulley comprises a first winding portion and a first mounting portion disposed on the first winding portion, the first perforation extends throughout the first winding portion and the first mounting portion, the first winding portion is located in the housing, the first mounting portion passes through the mounting hole, and the first matching portion and the first latching portion are arranged on the first mounting portion; and/or
the second cable pulley comprises a second winding portion and a second mounting portion disposed on the second winding portion, the second winding portion is located in the housing, the second mounting portion passes through the first perforation, and the second matching portion and the second latching portion are arranged on the second mounting portion.

20. An endoscope handle, comprising a first braking assembly, a second braking assembly, and the endoscope knob device according to any one of claims 1 to 19, wherein the first braking assembly is configured to brake the first knob or the first cable pulley, and the second braking assembly is configured to brake the second knob or the second cable pulley.

21. An endoscope, comprising the endoscope handle according to claim 20.
